# EUROPÄISCHE PATENTSCHRIFT

(11) **EP 3 817 641 B1**
(45) Veröffentlichungstag und Bekanntmachung des Hinweises auf die Patenterteilung: **27.10.2021**
(21) Anmeldenummer: 20737036.2
(22) Anmeldetag: 10.07.2020
(51) Int. Cl.: A61B 3/10

(54) **BESTIMMUNG EINER VERÄNDERUNG EINES REFRAKTIONSFEHLERS EINES AUGES**
DETERMINATION OF A CHANGE IN THE REFRACTION ERROR OF AN EYE
DÉTERMINATION D'UN CHANGEMENT D'UNE ERREUR DE RÉFECTION D'UN IL

(30) Priorität: 11.07.2019 EP 19185709
(43) Veröffentlichungstag der Anmeldung: 12.05.2021
(73) Patentinhaber: Carl Zeiss Vision International GmbH, 73430 Aalen (DE)
(72) Erfinder: OHLENDORF, Arne, 72074 Tübingen (DE); BREHER, Katharina, 72076 Tübingen (DE); WAHL, Siegfried, 73072 Donzdorf (DE)
(74) Vertreter: Altmann Stößel Dick Patentanwälte PartG mbB
(86) Internationale Anmeldenummer: PCT/EP2020/069566
(87) Internationale Veröffentlichungsnummer: WO 2021/005213

(56) Entgegenhaltungen:
- WO-A1-2015/188279
- WO-A1-2018/165697
- US-A1- 2017 172 405
- US-A1- 2019 033 619
- FLORES-MORENO IGNACIO ET AL: "The Relationship Between Axial Length and Choroidal Thickness in Eyes With High Myopia", AMERICAN JOURNAL OF OPHTHALMOLOGY, ELSEVIER, AMSTERDAM, NL, Bd. 155, Nr. 2, 1. Oktober 2012 (2012-10-01), Seite 314, XP028962937, ISSN: 0002-9394, DOI: 10.1016/J.AJO.2012.07.015
- MARY HO ET AL: "Choroidal Thickness Measurement in Myopic Eyes by Enhanced Depth Optical Coherence Tomography", OPHTHALMOLOGY, Bd. 120, Nr. 9, 1. September 2013 (2013-09-01), Seiten 1909-1914, XP055656379, AMSTERDAM, NL ISSN: 0161-6420, DOI: 10.1016/j.ophtha.2013.02.005

## Beschreibung

### Gebiet der Erfindung

Die vorliegende Erfindung betrifft ein Verfahren, eine Vorrichtung und ein Computerprogramm zur Bestimmung einer Veränderung eines Refraktionsfehlers eines Auges eines Nutzers.

### Stand der Technik

Aus dem Stand der Technik sind Verfahren zur Bestimmung von Refraktionsfehlern eines Auges eines Nutzers bekannt. Der Begriff "Refraktion" bezeichnet hierbei eine Lichtbrechung im Auge des Nutzers, die ein durch die Pupille in das Innere des Auges einfallender Lichtstrahl erfährt. Durch ein Vorhalten einer Anzahl von Brillengläsern mit bekannten Eigenschaften sowie durch eine Führung des Nutzers in einem Frageprozess kann subjektiv bestimmt werden, welche Defokussierung das Auge des Nutzers aufweist und welche zum Beispiel sphärozylindrische Ausgestaltung des Brillenglases zu einem weitgehenden Ausgleich der Refraktionsfehler des Auges und damit zu einer möglichst optimalen Bildqualität für den Nutzer führt. Die Defokussierung des Auges des Nutzers kann zu einer Fehlsichtigkeit (Ametropie) des Nutzers führen, insbesondere zu einer Kurzsichtigkeit (Myopie) oder zu einer Weitsichtigkeit (Hyperopie) des Nutzers. Vor allem in den asiatischen Ländern ist eine zunehmende Häufigkeit der Kurzsichtigkeit, insbesondere bei Kindern und Jugendlichen, zu beobachten, wobei die Kurzsichtigkeit in etwa 80 % der Fälle durch ein erhöhtes Augenlängenwachstum verursacht wird. Als Faustformel gilt hierbei, dass eine Verlängerung des Augapfels von etwa 1 mm zu einer Fehlsichtigkeit von ungefähr 3 D führt.

Weiterhin ist es bekannt, zur Bestimmung einer Veränderung des Refraktionsfehlers des Auges die Refraktion des Auges mit und ohne Zykloplegikum, d.h. Pharmazeutikum zur Reduktion der Akkommodationsfähigkeit des Auges, messtechnisch zu erfassen. Dies kann insbesondere zu einer Verlaufskontrolle von Fehlsichtigkeit oder zur Beobachtung einer Wirksamkeit von Ansätzen zur Verringerung der Progression der Fehlsichtigkeit erfolgen. Alternativ kann eine Achsenlänge des Auges, insbesondere von der Vorderfläche oder der Rückfläche der Hornhaut bis zum retinalen Pigmentepithel der Netzhaut, zum Beispiel mittels optischer Laserbiometrie über einen längeren Zeitraum erfasst werden und dieses Wachstum über diesen Zeitraum beobachtet werden. Hierzu kann eine Verlaufskontrolle über den Zeitraum von einem bis drei Jahren durchgeführt werden, wobei in der Regel mindestens zwei Messungen pro Jahr erfolgen. Allerdings sind auch andere Intervalle, zum Beispiel wenigstens eine wöchentliche, monatliche oder jährliche Messung, möglich. Damit liegt jedoch eine Aussage über die individuelle Progression oder die Wirksamkeit eines gewählten Ansatzes zur Reduktion der Progression erst nach Ablauf dieses Zeitraums vor.

Ferner ist es bekannt, dass ein Zusammenhang zwischen der Schichtdicke der Aderhaut des Auges und dessen Achsenlänge existiert. Zudem ist bekannt, dass die Aderhaut auf von außen induzierte dioptrische Reize mittels Dickenänderung reagieren kann, und dass die zeitliche Auflösung einer solchen Reaktion im Bereich von Minuten liegt. Zum Beispiel lassen sich Daten, die mittels optischer Kohärenztomographie (OCT) ermittelt werden, zur Bestimmung der Änderung der Schichtdicke der Aderhaut verwenden. Weitere optische Interventionsmöglichkeiten zur Reduzierung der Progression der Kurzsichtigkeit beruhen auf dioptrischen Theorien, zum Beispiel einer Unterakkommodations-Theorie oder einer peripheren Defokus-Theorie, wobei OCT-Messungen, insbesondere Spectral-Domain OCT (SD-OCT) und Swept-Source OCT, zur Bestimmung des Einflusses einer Interventionsmöglichkeit auf die Aderhautdicke verwendet werden können. Wird die Veränderung der Aderhautdicke mittels OCT ermittelt, kann eine Aussage über eine Verdünnung oder Verdickung der Aderhaut bereits nach mehreren Minuten oder Stunden erfolgen.

Allerdings weisen Messungen der Schichtdicke der Aderhaut im Allgemeinen eine zu geringe Wiederholbarkeit aus, um die in der Literatur beschriebene Effektgröße von circa 10 µm bis 30 µm bzw. bis zu 50 µm bei Primaten oder 260 µm bei Hühnern auch tatsächlich nachweisen zu können. Hinweise zu derartigen Messungen finden sich in D. Wang, R.K.M. Chun, M. Liu, R.P.K. Lee, Y. Sun, T. Zhang, C. Lam, Q. Liu und C.H. To1, Optical Defocus Rapidly Changes Choroidal Thickness in Schoolchildren, PLoS ONE 11(8): e0161535, doi: 10.1371/journal.pone. 0161535, 2016; S.A. Read, M.J. Collins, und B.P. Sander, Human Optical Axial Length and Defocus, Investigative Ophthalmology & Visual Science 51(12), S. 6262-69, 2010; S.T-H. Chiang, J.R. Phillips und S. Backhouse, Effect of retinal image defocus on the thickness of the human choroid, Ophthalmic Physiol. Opt, 35: 405-413. doi: 10.1111/ opo. 12218, 2015; D. Troilo, D.L. Nickla und C.F. Wildsoet, Choroidal Thickness Changes during Altered Eye Growth and Refractive State in a Primate, Investigative Ophthal-mology & Visual Science 41 (6), S. 1249-58, 2000; und J. Wallman, C. Wildsoet, A. Xu, M.D. Gottlieb, D.L. Nickla, L. Marran, W. Krebs, A.M. Christensen, Moving the Retina: Choroidal Modulation of Refractive State, Vision Res. 35(1), S. 37-50, 1995. WO 2018/165697 A1 offenbart Vorrichtungen und Verfahren zur Implementierung eines ophthalmologischen Linsendesigns. Nach erfolgter Implementierung kann der Einfluss des ophthalmologischen Linsendesigns mittels einer okularen biometrischen Vorrichtung gemessen werden. Insbesondere in Kombination einer adaptiven optischen Vorrichtung mit der okularen biometrischen Vorrichtung wurde der Einfluss des ophthalmologischen Linsendesigns auf die Schichtdicke der Aderhaut erfasst. Hierzu wurden Messungen an verschiedenen Positionen auf der Netzhaut (Retina) einschließlich der Sehgrube (Fovea) und an der retinalen Peripherie vorgenommen.

US 2019/033619 A1 offenbart ein Verfahren zum Überwachen und Hemmen eines Fortschreitens der Kurzsichtigkeit (Myopia) in einer Person, umfassend: Messen einer Änderung der Schichtdicke der Aderhaut über einen Zeitraum, insbesondere mittels optischer Kohärenztomographie (OCT); und Bereitstellen von ophthalmologischen Linsen, insbesondere in Form von Brillengläsern oder Kontaktlinsen, zur Verringerung eines Kontrasts bei peripherer Sicht im Vergleich zu dem Kontrast bei Sicht entlang der Achse des Auges. Die jeweils gewonnenen experimentellen Ergebnisse werden hierbei als Funktion der Position auf der Netzhaut dargestellt.

Flores-Moreno Ignacio et al., The Relationship Between Axial Length and Choroidal Thickness in Eyes With High Myopia, Am. J. Ophthalmology 155 (2), 2012, S. 314-319, offenbart Untersuchungen der Schichtdicke der Aderhaut in hochmyotropen Augen sowie deren Korrelation mit der axialen Länge des Auges, des Alters der Person und des sphärischen Äquivalents des Refraktionsfehlers. Die Schichtdicke der Aderhaut wurde vom äußeren Ende des retinalen Pigmentepithels bis zum Übergang zur Lederhaut (Sklera) in Intervallen von 500 µm bis 2500 µm nasal und temporal in Bezug auf die Fovea gemessen.

### Aufgabe der Erfindung

Insbesondere ausgehend von der Offenbarung in WO 2018/165697 A1 oder US 2019/ 033619 A1, besteht die Aufgabe der vorliegenden Erfindung darin, ein Verfahren, eine Vorrichtung und ein Computerprogramm zur Bestimmung einer Veränderung eines Refraktionsfehlers eines Auges eines Nutzers bereitzustellen, welche die aufgeführten Nachteile und Einschränkungen des Standes der Technik zumindest teilweise überwinden.

Insbesondere sollen es das vorliegende Verfahren, die Vorrichtung und das Computerprogramm ermöglichen, eine zuverlässige Bestimmung der Veränderung des Refraktionsfehlers des Auges, bevorzugt zur Klassifizierung eines Fortschreitens von Fehlsichtigkeit oder für eine Aussage über einen Erfolg einer Intervention zur Verringerung des Fortschreitens der Fehlsichtigkeit, durch Messung der Schichtdicke der Aderhaut bei einer hohen Wiederholbarkeit der Messergebnisse ermöglichen.

### Offenbarung der Erfindung

Diese Aufgabe wird gelöst durch ein Verfahren, ein Computerprogramm und eine Vorrichtung zur Bestimmung einer Veränderung eines Refraktionsfehlers eines Auges eines Nutzers mit den Merkmalen der unabhängigen Patentansprüche. Bevorzugte Ausgestaltungen, welche einzeln oder in Kombination realisierbar sind, sind in den abhängigen Ansprüchen dargestellt.

Im Folgenden werden die Begriffe "haben", "aufweisen", "umfassen" oder "einschließen" oder beliebige grammatikalische Abweichungen davon in nicht-ausschließlicher Weise verwendet. Dementsprechend können sich diese Begriffe sowohl auf Situationen beziehen, in welchen, neben dem durch diese Begriffe eingeführten Merkmal, keine weiteren Merkmale vorhanden sind oder auf Situationen, in welchen ein oder mehrere weitere Merkmale vorhanden sind. Beispielsweise kann sich der Ausdruck "A hat B", "A weist B auf, "A umfasst B" oder "A schließt B ein" sowohl auf die Situation beziehen, in welcher, abgesehen von B, kein weiteres Element in A vorhanden ist, d.h. auf eine Situation, in welcher A ausschließlich aus B besteht, als auch auf die Situation, in welcher, zusätzlich zu B, ein oder mehrere weitere Elemente in A vorhanden sind, beispielsweise Element C, Elemente C und D oder sogar weitere Elemente.

Der Refraktionsfehler eines Auges kann beispielsweise durch ein Brillenglas mittels sphärischer Korrektion und/oder astigmatischer Korrektion und der Korrektion der Achslage sowie optional mittels Korrektion durch ein Prisma mit Basislage bevorzugt weitestgehend ausgeglichen werden. Die vorstehend genannten Korrektionen können den Refraktionsfehler eines Auges für die Ferne und/oder für die Nähe bevorzugt weitestgehend ausgleichen.

Die vorliegende Erfindung versteht unter "Refraktionsfehler" alle Fehler eines Auges, welche mittels sphärischer Korrektion und/oder astigmatischer Korrektion und der Korrektion der Achslage sowie optional mittels Korrektion durch ein Prisma mit Basislage, jeweils für die Ferne, auszugleichen sind. Der Refraktionsfehler kann hierbei einen, einen Teil oder alle der vorstehend aufgeführten Fehler umfassen.

In einem ersten Aspekt betrifft die vorliegende Erfindung ein Verfahren zur Bestimmung einer Veränderung eines Refraktionsfehlers eines Auges eines Nutzers. Das Verfahren umfasst die folgenden Schritte a) und b), vorzugsweise in der angegebenen Reihenfolge, wobei eine ganz oder teilweise zeitgleiche Ausführung der Verfahrensschritte möglich ist. Weiterhin können Schritte dieses Verfahrens wiederholt, insbesondere mehr als einmal, ausgeführt werden. Das Verfahren kann, zusätzlich zu den genannten Verfahrensschritten auch weitere Schritte umfassen, unabhängig davon, ob diese in der vorliegenden Beschreibung erwähnt werden oder nicht. Hierzu kann insbesondere ein Schritt, welcher ein messtechnisches Erfassen einer Schichtdicke der Aderhaut des Auges des Nutzers umfasst, gehören.

Das Verfahren zur Bestimmung einer Veränderung eines Refraktionsfehlers eines Auges eines Nutzers, wobei das Auge des Nutzers eine Aderhaut aufweist, umfasst die Schritte:
a) Ermitteln mindestens eines Wertes für eine Schichtdicke der Aderhaut des Auges des Nutzers über mindestens einen Bereich der Aderhaut; und
b) Bestimmen eines Wertes für die Veränderung des Refraktionsfehlers des Auges ausschließlich aus mindestens zwei Werten für die Schichtdicke der Aderhaut, die jeweils zu unterschiedlichen Zeitpunkten für den mindestens einen Bereich der Aderhaut erfasst wurden, wobei der mindestens eine Bereich der Aderhaut aus einem nasalen perifovealen Bereich oder einem nasalen parafovealen Bereich der Aderhaut ausgewählt wird.

Die mit dem Verfahren bestimmbare Veränderung des Refraktionsfehlers umfasst bevorzugt die Korrektion für die Ferne, besonders bevorzugt die sphärische und astigmatische Korrektion sowie die Korrektion der Achslage, jeweils für die Ferne, ganz bevorzugt die sphärische Korrektion für die Ferne. Alternativ kann auch die Veränderung des sphärischen Äquivalents bestimmt werden.

Wie unten näher beschrieben, ermöglicht das vorliegende Verfahren zur Bestimmung der Veränderung des Refraktionsfehlers des Auges eines Nutzers insbesondere eine Klassifizierung eines Fortschreitens der Fehlsichtigkeit oder eine Aussage über einen Erfolg einer Intervention zur Verringerung des Fortschreitens der Fehlsichtigkeit, indem eine Messung der Schichtdicke der Aderhaut zu unterschiedlichen Zeitpunkten erfolgt, wobei die hiermit erzielten Messergebnisse - im Unterschied zu aus dem Stand der Technik bekannten Verfahren - eine hohe Wiederholbarkeit aufweisen. Mit dem vorliegenden Verfahren lässt sich so bereits in wenigen Minuten eine Veränderung des Refraktionsfehlers eines Auges mit hoher Aussagekraft bestimmen.

Gemäß Schritt a) des vorliegenden Verfahrens erfolgt ein Ermitteln mindestens eines Wertes für eine Schichtdicke der Aderhaut des Auges des Nutzers über mindestens einen Bereich der Aderhaut. Hierbei bezeichnen die Begriffe "Aderhaut" und "Choroidea" eine in der dem Lichteintritt abgewandten Seite des Auges zwischen der Lederhaut (Sclera) und der Netzhaut (Retina), insbesondere zwischen der Lederhaut und dem retinalen Pigmentepithel, angeordnete Zwischenschicht. Durch diese Art der Anordnung der Aderhaut im Auge ergibt sich, dass die Schichtdicke der Aderhaut durch die hieran jeweils angrenzende Lederhaut und Netzhaut, insbesondere der Lederhaut und dem retinalen Pigmentepithel, begrenzt wird. Der Begriff der "Schichtdicke der Aderhaut" bezeichnet im Rahmen der vorliegenden Erfindung einen Abstand zwischen der Lederhaut und der Netzhaut, wobei der Abstand im Wesentlichen senkrecht zu einer Richtung einer Ausdehnung der Aderhaut bestimmt wird. Hierbei kann die Ausdehnung der Aderhaut die Schichtdicke der Aderhaut um mindestens einen Faktor 10, bevorzugt mindestens einen Faktor 20, besonders bevorzugt mindestens einen Faktor 50, übersteigen. Für weitere Einzelheiten in Bezug auf die Anordnung der Aderhaut und die zugehörige Schichtdicke wird auf die untenstehende Beschreibung der Ausführungsbeispiele und die Figuren verwiesen.

Grundsätzlich kann dem Ermitteln des mindestens einen Wertes für die Schichtdicke der Aderhaut gemäß Schritt a) des vorliegenden Verfahrens die Erfassung der Schichtdicke der Aderhaut vorausgehen, die mit einem beliebigen Vorgehen und unter Verwendung einer beliebigen Vorrichtung, insbesondere einer unten näher beschriebenen Messeinrichtung, welche zur Erfassung der Schichtdicke der Aderhaut eingerichtet ist, erfolgen kann. Der Begriff der "Erfassung" bezieht sich hierbei auf eine Aufnahme mindestens einer messtechnisch erfassten Größe, aus der die Schichtdicke abgeleitet werden kann. Hierbei kann das Erfassen der Schichtdicke der Aderhaut des Auges bevorzugt ein Erzeugen einer räumlich aufgelösten Aufnahme des Auges umfassen, wobei die Aufnahme zumindest einen Bereich der Aderhaut und, vorzugsweise, einen hieran jeweils angrenzenden Bereich der Lederhaut und der Netzhaut, insbesondere dem retinalen Pigmentepithel, aufweist. Andere Arten des Erfassens der Schichtdicke der Aderhaut sind jedoch möglich.

In einer bevorzugten Ausgestaltung kann zum Erfassen der Schichtdicke der Aderhaut des Auges ein Verfahren, ausgewählt aus einem optischen Verfahren, einem akustischen Verfahren oder einem fotoakustischen Verfahren, eingesetzt werden, wobei, entsprechend dem jeweils ausgewählten Verfahren, eine optische Messeinrichtung, eine akustische Messeinrichtung oder eine fotoakustische Messeinrichtung Verwendung finden kann. Weitere Verfahren sind jedoch grundsätzlich möglich.

In einer besonders bevorzugten Ausgestaltung kann die Erfassung der Schichtdicke der Aderhaut mit einem optischen Verfahren erfolgen. Der Begriff des "optischen Verfahrens" bezeichnet hierbei eine Vorgehensweise, bei welcher insbesondere die Aderhaut des Auges mit Licht, vorzugsweise mit Licht aus dem sichtbaren oder dem infraroten Spektralbereich, beaufschlagt wird und woraufhin aus einer Reflexion des Lichtes an der Aderhaut auf diese Beaufschlagung mit Licht Rückschlüsse auf eine Beschaffenheit der Aderhaut, insbesondere auf die Schichtdicke der Aderhaut gezogen werden können. Im Gegensatz hierzu bezeichnen das "akustische Verfahren" und das "fotoakustische Verfahren" jeweils eine Vorgehensweise, bei welcher die Aderhaut mit akustischen Wellen, bevorzugt Ultraschall, beaufschlagt wird, wobei die messtechnische Erfassung ebenfalls akustisch oder optisch erfolgt.

In einer besonders bevorzugten Ausgestaltung kann das optische Verfahren ausgewählt werden aus einem Verfahren zur optischen Kohärenztomographie. Hierbei bezeichnet der Begriff der "optischen Kohärenztomographie", abgekürzt auch "OCT", ein bildgebendes Verfahren zur Erzeugung einer zwei-dimensionalen oder einer drei-dimensionalen Aufnahme eines biologischen Gewebes, insbesondere der Aderhaut des Auges und hieran angrenzender Schichten, wobei bevorzugt eine Auflösung im Mikrometerbereich erhalten werden kann. Zur Erzeugung der gewünschten Aufnahme wird Licht zeitlich geringer Kohärenzlänge aus einer Strahlungsquelle in einem Strahlteiler in zwei Teile geteilt, wobei ein erster Teil des Lichts das Gewebe entlang einer optischen Achse beaufschlagt, wobei ein zweiter Teil des Lichts über eine Referenzstrecke geführt wird, und wobei das von dem Gewebe reflektierte Licht mit dem über die Referenzstrecke geführten Referenzlicht interferiert wird, um ein Interferenzsignal zu generieren. Aus dem so generierten Interferenzsignal lassen sich zunächst nur Strukturen in dem Gewebe entlang der optischen Achse unterscheiden; allerdings kann durch laterales Scannen mittels Ändern der optischen Achse über das Gewebe die gewünschte zwei- oder dreidimensionale Aufnahme des Gewebes oder eines Ausschnitts hiervon erzeugt werden.

Vorzugsweise kann das Verfahren der optischen Kohärenztomographie ausgewählt werden aus einer Fourier-Domain OCT oder einer Time-Domain OCT, wobei die Fourier-Domain OCT, insbesondere eine Spectral-Domain OCT oder eine Swept-Source OCT, besonders bevorzugt sind. Der Begriff der "Time-Domain OCT" bezeichnet hierbei ein Vorgehen, bei welchem eine Länge der Referenzstrecke verändert und hierbei kontinuierlich eine Intensität der Interferenz erfasst wird, wobei eine Veränderung des Frequenzspektrums der Interferenz außer Betracht bleibt. Demgegenüber bezeichnet die "Fourier-Domain OCT" ein Vorgehen, bei welchem die Veränderung von Komponenten des Frequenzspektrums der Interferenz berücksichtigt wird. Wird die Veränderung des Frequenzspektrums der Interferenz mittels einer breitbandigen Strahlungsquelle gleichzeitig angeregt und erfasst, so wird dieses Vorgehen als "Spectral-Domain OCT" bezeichnet. Bei der "Swept-Source OCT" werden dagegen die Komponenten des Frequenzspektrums, insbesondere durch sukzessive Abstimmung der Frequenzanregung der Strahlungsquelle, zeitlich aufeinanderfolgend angeregt und erfasst.

In einer alternativen Ausgestaltung kann das optische Verfahren ausgewählt werden aus einem adaptiven optischen Verfahren. Der Begriff des "adaptiven optischen Verfahrens" bezeichnet hierbei ein optisches Verfahren, in welchem ein abbildendes optisches System, das einen Strahlengang mit Abbildungseigenschaften umfasst, dazu eingerichtet ist, optische Veränderungen, die in dem Strahlengang auftreten, durch eine Änderung der Abbildungseigenschaften zu kompensieren, wobei es möglich ist, aus der Änderung der Abbildungseigenschaften Rückschlüsse auf optische Veränderungen in dem Strahlengang zu ziehen. Dadurch kann erfindungsgemäß auf eine Schichtdicke der Aderhaut geschlossen werden, wenn die Aderhaut derart in das optische System integriert wird, dass sich eine Veränderung der Schichtdicke der Aderhaut die Abbildungseigenschaften des optischen Systems ändert. Bevorzugt kann das adaptive optische Verfahren ausgewählt sein aus einem Verfahren zur Bestimmung einer optischen Transmission oder einem Verfahren zur Bestimmung einer optischen Reflektivität, so dass eine Veränderung der optischen Transmission oder Reflektivität der Aderhaut, bevorzugt mittels einer so genannten "Fundusreflektivität", die Abbildungseigenschaften des optischen Systems ändert und dadurch die gewünschte Erfassung der Schichtdicke der Aderhaut ermöglicht. Andere Arten von adaptiven optischen Verfahren und Einrichtungen sind jedoch denkbar.

Gemäß Schritt a) erfolgt weiterhin ein Ermitteln des mindestens eines Wertes für die Schichtdicke der Aderhaut. Der Begriff des "Ermitteln" bezieht sich hierbei auf die Bestimmung eines Wertes für die Schichtdicke der Aderhaut aus mindestens einer messtechnisch erfassten Größe, aus der die Schichtdicke abgeleitet werden kann. Hierbei kann das Ermitteln des Wertes für die Schichtdicke der Aderhaut vorzugsweise, insbesondere wenn ein Verfahren der optischen Kohärenztomographie ausgewählt wird, die Bestimmung des Wertes für die Schichtdicke aus einer räumlich aufgelösten Aufnahme des Auges umfassen, wobei die Aufnahme zumindest einen Bereich der Aderhaut und, bevorzugt, einen hieran jeweils angrenzenden Bereich der Lederhaut und der Netzhaut, insbesondere dem retinalen Pigmentepithel, aufweist. Andere Arten des Ermittelns der Schichtdicke der Aderhaut sind jedoch möglich, insbesondere wenn ein oben beschriebenes adaptives optisches Verfahren zur Erfassung der Schichtdicke eingesetzt wird.

Gemäß Schritt a) erfolgt das Ermitteln des mindestens einen Wertes für die Schichtdicke der Aderhaut über mindestens einen Bereich der Aderhaut. Der mindestens eine Bereich der Aderhaut bezeichnet hierbei einen Abschnitt der Aderhaut, der, wie unten näher erläutert, bevorzugt in Bezug auf eine Stelle der hieran angrenzenden Netzhaut ausgewählt werden kann. Hierbei kann über den mindestens einen Bereich der Aderhaut vorzugsweise ein Wert für eine durchschnittliche Schichtdicke der Aderhaut ermittelt werden. Die "durchschnittliche Schichtdicke" bezeichnet hierbei einen Mittelwert, bevorzugt einen arithmetischen oder einen geometrischen Mittelwert, einen Median oder einen anderen geeigneten repräsentativen Wert für die Schichtdicke der Aderhaut über den mindestens einen Bereich. Die "durchschnittliche Schichtdicke" bezeichnet besonders bevorzugt einen arithmetischen Mittelwert. Ein anderer Wert für die Schichtdicke der Aderhaut über den mindestens einen Bereich der Aderhaut ist jedoch denkbar.

Gemäß Schritt b) erfolgt, bevorzugt in einer unten näher beschriebenen Auswerteinrichtung, vorzugsweise im Anschluss an das Ermitteln des mindestens eines Wertes für die Schichtdicke der Aderhaut über den mindestens einen Bereich der Aderhaut entsprechend Schritt a), eine Bestimmung eines Wertes für die Veränderung des Refraktionsfehlers des Auges des Nutzers. Hierzu werden erfindungsgemäß ausschließlich mindestens zwei Werte für die Schichtdicke der Aderhaut verwendet, welche jeweils zu unterschiedlichen Zeitpunkten gemäß Schritt a) für den mindestens einen Bereich der Aderhaut ermittelt wurden. Bevorzugt werden hierzu zu einem ersten Zeitpunkt ein erster Wert für die Schichtdicke der Aderhaut und zu einem zweiten Zeitpunkt ein zweiter Wert für die Schichtdicke der Aderhaut ermittelt, wobei aus einem Inbeziehungsetzen der beiden Werte, d.h. des ersten Wertes und des zweiten Wertes, der gewünschte Wert für die Veränderung des Refraktionsfehlers des Auges des Nutzers ermittelt werden kann. Hierzu kann zum Beispiel aus der Veränderung der Schichtdicke, die sich aus einer Differenz zwischen dem ersten Wert für die Schichtdicke der Aderhaut und dem zweiten Wert für die Schichtdicke der Aderhaut ermitteln lässt, ein Wert für eine Verlängerung des Augapfels des Nutzers abschätzen. Bei Verwendung der eingangs genannten Faustformel, gemäß welcher eine Verlängerung des Augapfels von etwa 1 mm zu einer Fehlsichtigkeit von ungefähr 3 D führt, lässt sich somit auf die Veränderung des Refraktionsfehlers des Auges des Nutzers schließen. Andere Arten der Bestimmung des Wertes für die Veränderung des Refraktionsfehlers des Auges des Nutzers sind jedoch möglich.

Erfindungsgemäß wird gemäß Schritt b) zur Bestimmung des Wertes für die Veränderung des Refraktionsfehlers der mindestens eine Bereich der Aderhaut aus einem nasalen perifovealen Bereich oder einem nasalen parafovealen Bereich der Aderhaut ausgewählt, wobei der nasale perifoveale Bereich der Aderhaut besonders bevorzugt ist. Der mindestens eine Bereich der Aderhaut bezeichnet hierbei einen Abschnitt der Aderhaut, insbesondere in Bezug auf eine Stelle der hieran angrenzenden Netzhaut, vorzugsweise in Bezug auf die Sehgrube (Fovea oder Fovea centralis), welche eine sich im Zentrum des Gelben Flecks (Macula lutea) befindliche Einsenkung der Netzhaut des Auges umfasst, welche bei Mensch und Säugetieren als Bereich höchster Sehschärfe gilt.

In einer besonders bevorzugten Ausgestaltung kann eine Fläche der Netzhaut und damit der hieran angrenzenden Aderhaut in einen subfovealen Bereich, den subfovealen Bereich umgebende parafoveale Bereiche oder innere Bereiche und die parafovealen Bereiche umgebende perifoveale Bereiche oder äußeren Bereiche aufgeteilt werden. Hierbei kann der subfoveale Bereich vorzugsweise einen Durchmesser von 0,5 mm bis 1,5 mm, bevorzugt 1 mm, entsprechend einem Winkel von 3,33°, aufweisen, während die parafovealen Bereiche sich bis hieran anschließend bis zu einem Innendurchmesser und die perifovealen Bereiche sich hieran anschließend bis zu einem Außendurchmesser erstrecken können, wobei der Innendurchmesser vorzugsweise ausgewählt wird aus einem Wert von 2 mm bis 4,5 mm, bevorzugt 2,5 mm bis 4 mm, insbesondere 3 mm, entsprechend einem Winkel von 10°, und der Außendurchmesser ausgewählt wird aus einem Wert von 5 mm bis 20 mm, bevorzugt 5 mm bis 12 mm, insbesondere 6 mm, entsprechend einem Winkel von 20°. Andere Werte für die hier genannten Durchmesser sind jedoch denkbar.

Wie bereits erwähnt, erfolgt erfindungsgemäß die Bestimmung des Wertes für die Veränderung des Refraktionsfehlers gemäß Schritt b) durch Verwenden der mindestens zwei Werte für die Schichtdicke der Aderhaut, die für einen nasalen perifovealen Bereich oder einen nasalen parafovealen Bereich der Aderhaut ermittelt wurden. Wie aus den unten stehenden Ausführungsbeispielen und den Figuren hervorgeht, eignen sich hierzu besonders bevorzugt ein äußerer nasaler Bereich und/oder ein äußerer temporaler Bereich der Netzhaut, insbesondere der mit "7" (rechtes Auge) und "9" (linkes Auge) bezeichnete Bereich gemäß einem so genannten "ETDRS-Gitter" der Netzhaut. Das ETDRS-Gitter der Netzhaut bezieht sich auf ein so genanntes "Early Treatment Diabetic Retinopathy Study (ETDRS) Grid", wie es zum Beispiel im CIRRUS HD-OCT Benutzerhandbuch, 2660021164328 Rev. A 2016-10, Anhang A-7, zu finden ist. Hierin wird die Netzhaut in Bereiche von 1 bis 9 in Bezug auf die Lage der Fovea aufgeteilt.

Während bekannte Verfahren zur Bestimmung der Veränderung der Schichtdicke der Aderhaut etwaige Veränderungen der Schichtdicke der Aderhaut vor allem im subfovealen Bereich der Netzhaut auswerten, konnte experimentell überraschenderweise nachgewiesen werden (siehe Tabelle 1 unten), dass die Wiederholbarkeit der Auswertung in den nasalen perifovealen Bereichen, d.h. in den Bereichen 7 und 9 im ETDRS-Gitter, und in dem nasalen parafovealen Bereich, d.h. in Bereich 3 für das rechte Auge und in Bereich 5 für das linke Auge im ETDRS-Gitter, insbesondere jedoch in dem nasalen perifovealen Bereich, d.h. in dem Bereich 7 für das rechte Auge und in dem Bereich 9 für das linke Auge, signifikant besser ist als im subfovealen Bereich der Netzhaut und in den anderen Bereichen der Aderhaut. Damit ermöglicht das vorliegende Verfahren die gewünschte zuverlässige Bestimmung der Veränderung des Refraktionsfehlers des Auges bei einer hohen Wiederholbarkeit der Messergebnisse und erlaubt daher bevorzugt eine zuverlässigere Klassifizierung des Fortschreitens von Fehlsichtigkeit und zuverlässigere Aussagen über den Erfolg einer Intervention zur Verringerung des Fortschreitens der Fehlsichtigkeit.

In einem weiteren Aspekt betrifft die vorliegende Erfindung ein Computerprogramm zur Bestimmung eines Refraktionsfehlers eines Auges eines Nutzers, wobei das Computerprogramm dazu eingerichtet ist, die Bestimmung des Refraktionsfehlers des Auges des Nutzers gemäß dem hierin beschriebenen Verfahren zur Bestimmung eines Refraktionsfehlers eines Auges eines Nutzers durchzuführen.

Das hier vorgeschlagene Verfahren zur Bestimmung einer Veränderung eines Refraktionsfehlers eines Auges eines Nutzers eignet sich auch zur Verwendung in einem Verfahren zur Herstellung eines Brillenglases für das Auge des betreffenden Nutzers. Unter einem "Brillenglas" wird gemäß Norm DIN EN ISO 13666:2013-10, Abschnitte 8.1.1 und 8.1.2 eine optische Linse verstanden, die im Rahmen der vorliegenden Erfindung zur Korrektion von Fehlsichtigkeit des Auges dienen soll, wobei die optische Linse vor dem Auge des Nutzers, aber nicht in Kontakt mit dem Auge getragen wird. Der Begriff der "Brille" bezeichnet ein beliebiges Element, das zwei einzelne Brillengläser und eine Brillenfassung umfasst, wobei das Brillenglas zur Einbringung in eine Brillenfassung vorgesehen ist, die von einem Nutzer der Brille ausgewählt wird. Insbesondere kann aus einer Bestimmung der bei dem Nutzer auftretenden Veränderung des Refraktionsfehlers eine sphärozylindrische Linse ermittelt werden, die als Brillenglas dazu benutzt wird, die als Defokussierung des Auges auftretenden Refraktionsfehler derart zu kompensieren, dass eine möglichst optimale Bildqualität für den Nutzer erzielt werden kann.

In einem weiteren Aspekt betrifft die vorliegende Erfindung daher ein Verfahren zur Herstellung eines Brillenglases, wobei die Herstellung des Brillenglases durch Bearbeitung eines Brillenglasrohlings erfolgt, wobei der Brillenglasrohling anhand von Korrektionsdaten und Zentrierdaten bearbeitet wird, wobei die Zentrierdaten Anweisungen zur Positionierung des Brillenglases vor dem Auge eines Nutzers zum Ausgleich des Refraktionsfehlers des Auges des Nutzers umfassen, wobei eine Bestimmung einer Änderung des Refraktionsfehlers des Auges des Nutzers gemäß dem hierin beschriebenen Verfahren zur Bestimmung einer Veränderung eines Refraktionsfehlers eines Auges eines Nutzers erfolgt. Das Verfahren zur Herstellung eines Brillenglases wird insbesondere dann eingesetzt, wenn die Veränderung des Refraktionsfehlers des Auges des Nutzers einen Schwellwert, vorzugsweise ausgewählt aus einem Intervall von 0,25 D bis 1,5 D, insbesondere 0,25 D; 0,5 D; 0,75 D; 1 D; 1,25 D oder 1,5 D, erreicht oder überschreitet.

In einem weiteren Aspekt betrifft die vorliegende Erfindung eine Vorrichtung zur Bestimmung der Veränderung des Refraktionsfehlers des Auges des Nutzers, wobei das Auge des Nutzers eine Aderhaut aufweist. Erfindungsgemäß umfasst die Vorrichtung
- eine Messeinrichtung zum Erfassen einer Schichtdicke der Aderhaut des Auges des Nutzers; und
- eine Auswerteinrichtung zum Ermitteln von Werten für die Schichtdicke der Aderhaut des Auges über mindestens zwei Bereiche der Netzhaut des Auges, wobei die Auswerteinrichtung weiterhin zum Bestimmen eines Wertes für die Veränderung des Refraktionsfehlers des Auges ausschließlich aus mindestens zwei Werten für die Schichtdicke der Aderhaut eingerichtet ist, wobei die Werte für die Schichtdicke der Aderhaut jeweils zu unterschiedlichen Zeitpunkten für den mindestens einen Bereich der Aderhaut ermittelt werden, wobei der mindestens eine Bereich der Aderhaut aus einem nasalen perifovealen Bereich oder einem nasalen parafovealen Bereich der Aderhaut ausgewählt wird.

Insbesondere zum Ermitteln von Werten für die Schichtdicke der Aderhaut des Auges und zum Bestimmen des Wertes für die Veränderung des Refraktionsfehlers des Auges kann, vorzugsweise in der Auswerteinrichtung, eine automatisierte Software eingesetzt werden. Alternativ sind eine semi-automatisierte Auswertung mit manueller Korrektur durch einen Untersucher oder eine vollständige manuell Auswertung durch den Untersucher mittels einer Bestimmung von Grenzen zwischen dem retinalen Pigmentepithel der Netzhaut und der Aderhaut, sowie zwischen der Aderhaut und der Lederhaut bestimmt. Hierzu kann bekannte Software verwendet werden, welche eine Segmentierung jedes Scanbilds eines OCTs basierend auf Helligkeitsunterschieden zwischen benachbarten Pixeln basierend auf einer so genannten "Graph-Search-Methodik" durchführen. Alternativ sind grundsätzlich auch Algorithmen aus den Bereichen Machine Vision, Machine Learning oder Künstliche Intelligenz anwendbar, insbesondere um die Aderhaut zu segmentieren und um Längen, Flächen oder Volumina zu erfassen.

Die Auswerteinrichtung kann eine beliebige Einrichtung sein, die zu einem Empfang, einer Verarbeitung und einer Ausgabe von Daten eingerichtet ist. Hierbei kann es sich vorzugsweise um eine elektronisch oder optoelektronisch steuerbare Messeinheit handeln, die insbesondere über einen Computer, Mikrocomputer oder programmierbaren Chip, z.B. eine anwendungsspezifische integrierte Schaltung (engl. *application-specific integrated circuit;* ASIC) oder ein FPGA (engl*. field-programmable gate array),* verfügen kann, wobei die Messeinrichtung auf ein oder mehrere Computerprogramme, welche zur Durchführung der hierin beschriebenen Verfahren eingerichtet sein können, zugreifen kann. Alternativ kann die Auswerteinrichtung durch ein mobiles Kommunikationsgerät umfasst sein, insbesondere einem Smartphone oder einem Tablet. Andere Ausgestaltungen der Auswerteinrichtung sind jedoch möglich, zum Beispiel eine Integration der Auswerteinrichtung in die genannte Messeinrichtung.

Für Definitionen und optionale Ausgestaltungen des Computerprogramms und der Vorrichtung zur Bestimmung einer Veränderung eines Refraktionsfehlers eines Auges eines Nutzers wird auf die oben oder unten stehende Beschreibung des Verfahrens zur Bestimmung eines Refraktionsfehlers eines Auges eines Nutzers verwiesen.

### Kurze Beschreibung der Figuren

Weitere Einzelheiten und Merkmale der Erfindung ergeben sich aus der nachfolgenden Beschreibung von bevorzugten Ausführungsbeispielen, insbesondere in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktionen einander entsprechende Elemente. Im Einzelnen zeigen:
- Figur 1: ein bevorzugtes Ausführungsbeispiel einer Vorrichtung zur Bestimmung einer Veränderung eines Refraktionsfehlers eines Auges eines Nutzers;
- Figur 2: eine schematische Darstellung einer Lage der Aderhaut zwischen Lederhaut und Netzhaut;
- Figur 3: eine schematische Darstellung einer Einteilung der Netzhaut in verschiedene Bereiche; und
- Figur 4: ein Ablaufdiagramm des erfindungsgemäßen Verfahrens zur Bestimmung der Veränderung des Refraktionsfehlers des Auges des Nutzers.

### Ausführungsbeispiele

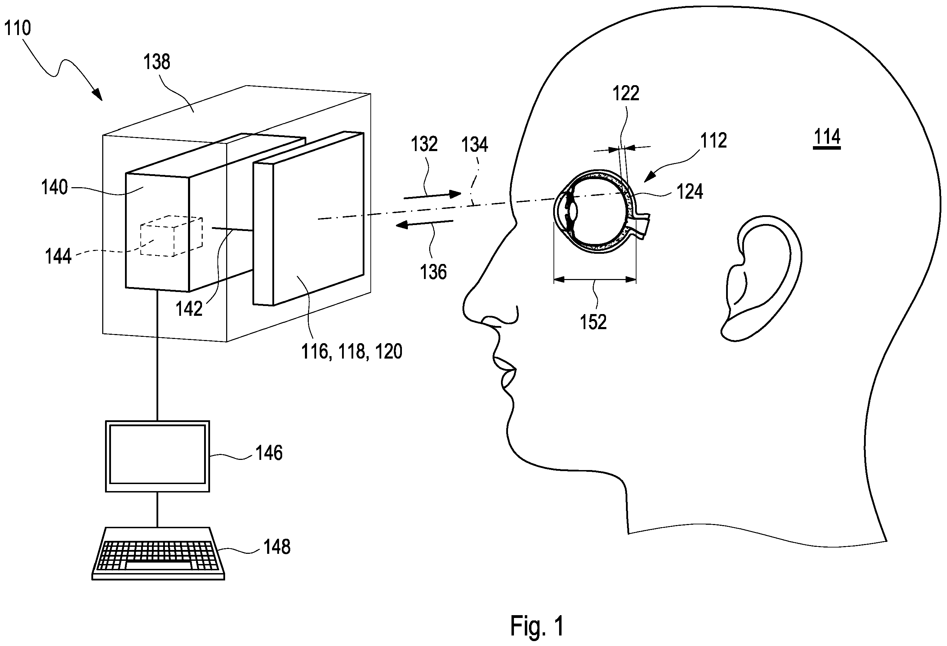

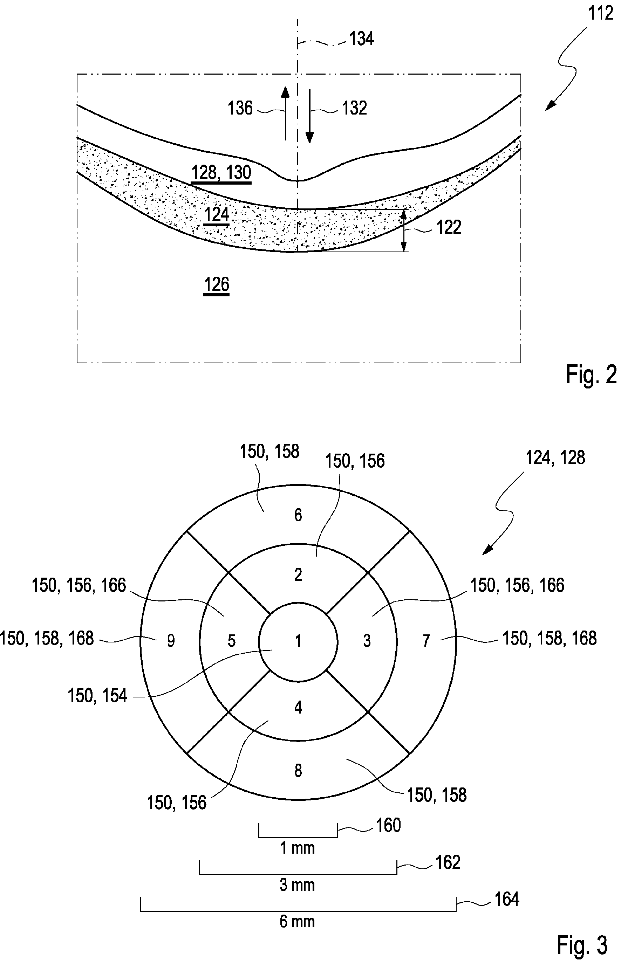

Figur 1 zeigt schematisch ein bevorzugtes Ausführungsbeispiel einer Vorrichtung 110 zur Bestimmung einer Veränderung eines Refraktionsfehlers eines Auges 112 eines Nutzers 114. Die Vorrichtung 110 umfasst eine Messeinrichtung 116, vorzugsweise eine optische Messeinrichtung 118, insbesondere eine Einrichtung 120 zur optischen Kohärenztomographie, die zum Erfassen einer Schichtdicke 122 der Aderhaut 124 des Auges 112 des Nutzers 114 eingerichtet ist. Andere Arten von Messeinrichtungen, insbesondere akustische oder fotoakustische Messeinrichtungen, sind jedoch denkbar. Wie in Figur 2 schematisch dargestellt, stellt die Aderhaut (Choroidea) 124 eine Zwischenschicht dar, die auf der dem Lichteintritt abgewandten Seite des Auges 112 zwischen Lederhaut (Sclera) 126 und Netzhaut (Retina) 128, insbesondere zwischen der Lederhaut 126 und dem retinalen Pigmentepithel 130, angeordnet ist.

Die Einrichtung 120 zur optischen Kohärenztomographie ermöglicht ein bildgebendes Verfahren zur Erzeugung einer 2- oder 3-dimensionalen Aufnahme eines biologischen Gewebes, welches hier die Aderhaut 124 und die hieran im Auge 112 angrenzenden Schichten Lederhaut 126 und retinales Pigmentepithel 130 umfasst, wobei bevorzugt eine Auflösung im Mikrometerbereich erhalten werden kann. Zur Erzeugung der gewünschten Aufnahme wird Licht 132 zeitlich geringer Kohärenzlänge aus einer Strahlungsquelle in einem Strahlteiler in zwei Teile geteilt, wobei ein erster Teil des Lichts das Gewebe entlang einer optischen Achse 134 beaufschlagt, wobei ein zweiter Teil des Lichts über eine Referenzstrecke geführt wird, und wobei das von dem Gewebe reflektierte Licht 136 mit dem über die Referenzstrecke geführten Referenzlicht interferiert wird, um ein Interferenzsignal zu generieren. Aus dem Interferenzsignal lassen sich zunächst nur Strukturen in dem Gewebe entlang der optischen Achse 134 unterscheiden; allerdings kann durch laterales Scannen mittels Ändern der optischen Achse 134 über das Gewebe die gewünschte 2- oder 3-dimensionale Aufnahme des Gewebes oder eines Ausschnitts hiervon erzeugt werden.

In der vorliegenden Ausführung kann das Verfahren zur optischen Kohärenztomographie bevorzugt ausgewählt werden aus einer Fourier-Domain OCT, insbesondere einer Spectral-Domain OCT oder Swept-Source OCT. Alternativ ist eine Time-Domain OCT ebenfalls denkbar. Eine weitere alternative Ausgestaltung kann eine Einrichtung zur Durchführung eines adaptiven optischen Verfahrens (nicht dargestellt) umfassen, die zur Durchführung eines adaptiven optischen Verfahrens eingerichtet ist, insbesondere eines Verfahrens zur Bestimmung einer optischen Transmission oder Reflektivität der Aderhaut 124.

Wie in Figur 1 schematisch dargestellt, kann die Vorrichtung 110 weiterhin ein Gehäuse 138 aufweisen, welches zusätzlich zu der Messeinrichtung 116 eine Auswerteinrichtung 140 umfassen kann. Alternativ oder zusätzlich kann die Auswerteinrichtung 140 jedoch auch außerhalb des Gehäuses 138 angebracht sein, wobei eine drahtgebundene oder eine drahtlose Verbindung 142 zwischen der Messeinrichtung 116 und der Auswerteinrichtung 140 vorhanden sein kann. Die Auswerteinrichtung 140 kann über einen Prozessor 144 verfügen, der zur Durchführung eines oder mehrerer Algorithmen vorgesehen sein kann. Weiterhin können ein Monitor 146 zur Darstellung von Werten und eine Tastatur 148 zur Eingabe von Werten oder Befehlen vorgesehen sein. Weitere Arten der Ausführung der Vorrichtung 110, insbesondere der Auswerteinrichtung 140, sind jedoch möglich.

Die Auswerteinrichtung 140 ist zum Ermitteln von Werten für die Schichtdicke 122 der Aderhaut 124 des Auges 112 über mindestens einen Bereich 150 der Aderhaut eingerichtet. Hierzu kann die Auswerteinrichtung 140 messtechnisch erfasste Größen empfangen, die von der Messeinrichtung 116 über die Verbindung 142 an die Auswerteinrichtung 140 weiter geleitet werden, wobei aus den messtechnisch erfassten Größen die Schichtdicke 122 der Aderhaut 124 des Auges 112 abgeleitet werden kann.

Erfindungsgemäß ist die Auswerteinrichtung weiterhin zum Bestimmen eines Wertes für die Veränderung des Refraktionsfehlers des Auges 112 ausschließlich aus mindestens zwei Werten für die Schichtdicke 122 der Aderhaut 124 eingerichtet ist, wobei die beiden Werte für die Schichtdicke 122 der Aderhaut 124 jeweils zu unterschiedlichen Zeitpunkten für den mindestens einen Bereich 150 der Aderhaut ermittelt werden. Der mindestens eine Bereich 150 der Aderhaut 124 bezeichnet hierbei einen Abschnitt der Aderhaut 124, der bevorzugt in Bezug auf eine Stelle der hieran angrenzenden Netzhaut 128 ausgewählt werden kann. Hierbei kann für den mindestens einen Bereich 150 der Aderhaut 124 bevorzugt ein Wert für eine durchschnittliche Schichtdicke 122 der Aderhaut 124 ermittelt werden. Andere Arten von Werten sind jedoch möglich.

Bevorzugt werden zu einem ersten Zeitpunkt ein erster Wert für die Schichtdicke 122 der Aderhaut 124 und zu einem zweiten Zeitpunkt ein zweiter Wert für die Schichtdicke 122 der Aderhaut 124 ermittelt, wobei der gewünschte Wert für die Veränderung des Refraktionsfehlers des Auges 112 des Nutzers 114 aus einem Inbeziehungsetzen des ersten Wertes und des zweiten Wertes ermittelt werden kann. Hierzu kann zum Beispiel aus der Veränderung der Schichtdicke 122, die sich aus einer Differenz zwischen dem ersten Wert für die Schichtdicke 122 und dem zweiten Wert für die Schichtdicke 122 ermitteln lässt, ein Wert für eine Änderung einer Länge 152 des Augapfels des Nutzers 114 abschätzen. Bei Verwendung der eingangs genannten Faustformel, gemäß welcher eine Zunahme der Länge 152 des Augapfels des Nutzers 114 von etwa 1 mm zu einer Fehlsichtigkeit von ungefähr 3 D führt, lässt sich somit auf die Veränderung des Refraktionsfehlers des Auges 112 des Nutzers 114 schließen.

Figur 3 zeigt die verschiedenen Bereiche 150 eines so genannten "ETDRS-Gitters" der Netzhaut 128, die mit den Ziffern "1" bis "9" versehen sind. Herbei werden ein subfovealer Bereich 154 mit der Ziffer "1" und den subfovealen Bereich 154 umgebende parafoveale Bereiche 156 mit den Ziffern "2", "3", "4" und "5" von den die parafovealen Bereiche 156 umgebenden perifovealen Bereiche 156 mit den Ziffern "6", "7", "8" und "9" unterschieden. In einer besonders bevorzugten Ausgestaltung kann eine Fläche der Netzhaut 128 und damit der hieran angrenzenden Aderhaut 124 in einen subfovealen Bereich 154, den subfovealen Bereich umgebende parafoveale Bereiche 156 oder innere Bereiche und die parafovealen Bereiche umgebende perifoveale Bereiche 158 oder äußeren Bereiche aufgeteilt werden. Hierbei kann der subfoveale Bereich 154 vorzugsweise einen Durchmesser 160 von 0,5 mm bis 1,5 mm, bevorzugt 1 mm, entsprechend einem Winkel von 3,33°, aufweisen, während die parafovealen Bereiche 156 sich bis hieran anschließend zu einem Innendurchmesser 162 und die sich hieran anschließenden perifovealen Bereiche 158 bis zu einem Außendurchmesser 164 erstrecken können, wobei der Innendurchmesser 162 vorzugsweise ausgewählt werden kann aus einem Wert von 2,5 mm bis 4,5 mm, bevorzugt 2,5 mm bis 4 mm, insbesondere 3 mm, d.h. entsprechend einem Winkel von 10°, und der Außendurchmesser 164 bevorzugt ausgewählt werden kann aus einem Wert von 5 mm bis 20 mm, bevorzugt 5 mm bis 12 mm, insbesondere 6 mm, d.h. entsprechend einem Winkel von 20°. Andere Werte für die hier genannten Durchmesser 160, 162, 164 sind jedoch denkbar.

Erfindungsgemäß wird der mindestens eine Bereich 150 der Aderhaut 124, der zur Bestimmung der Veränderung des Refraktionsfehlers des Auges 112 des Nutzers 114 verwendet wird, aus einem nasalen perifovealen Bereich 168 oder einem nasalen parafovealen Bereich 166 der Aderhaut 124 ausgewählt. Während bekannte Verfahren zur Bestimmung der Veränderung der Schichtdicke der Aderhaut etwaige Veränderungen der Schichtdicke 122 der Aderhaut 124 vor allem im subfovealen Bereich 154 der Netzhaut 128 auswerten, konnte experimentell überraschenderweise nachgewiesen werden, dass eine Wiederholbarkeit der Auswertung in dem nasalen perifovealen Bereich 168 und in dem nasalen parafovealen Bereich 166, d.h. den Bereichen "3" und "7" für das rechte Auge und den Bereichen "5" und "9" für das linke Auge in dem ETDRS-Gitter, insbesondere in dem nasalen perifovealen Bereich 168, d.h. dem Bereich "7" für das rechte Auge und dem Bereich "9" für das linke Auge in dem ETDRS-Gitter, signifikant besser ist als im subfovealen Bereich 154, in den übrigen, d.h. nicht-nasalen parafovealen Bereichen, d.h. den Bereichen "2" und "4" in dem ETDRS-Gitter, und in den übrigen, d.h. nicht-nasalen perifovealen Bereichen, d.h. den Bereichen "6" und "8" in dem ETDRS-Gitter.

Untersuchungen unter Verwendung der Spectral-Domain OCT als Messverfahren zur Erfassung der Schichtdicke 122 der Aderhaut 124 haben gezeigt, dass die beste Wiederholbarkeit vor allem in dem nasalen parafovealen Bereich 166 sowie in dem nasalen perifovealen Bereich 168, insbesondere in dem nasalen perifovealen Bereich 168, auftritt. Tabelle 1 umfasst Messwerte für die Wiederholbarkeit von Messungen zur Erfassung der Schichtdicke 122 der Aderhaut 124, aufgelistet nach den einzelnen Bereichen 150 der Aderhaut 124:

| **Tabelle 1** | |
|---|---|
| Bereich 150 der Aderhaut 124 | Wiederholbarkeit |
| subfovealer Bereich 154 | > 40 µm, im Mittel 60 µm |
| alle parafovealen Bereiche 156 | > 20 µm, im Mittel 30 µm |
| alle perifovealen Bereiche 158 | > 15 µm, im Mittel 30 µm |
| nasaler parafovealer Bereich 166 | > 15 µm, im Mittel 25 µm |
| nasaler perifovealer Bereich 168 | > 10 µm, im Mittel 20 µm |

Somit bietet der nasale perifoveale Bereich 168 die genaueste Wiederholbarkeit innerhalb der Aderhaut. Grundsätzlich zeigt sich, belegt durch die in Tabelle 1 aufgeführten Werte, dass die Wiederholbarkeit in jedem Bereich 150 der Aderhaut 124 besser ist, je entfernter der betreffende Bereich 150 von dem subfovealen Bereich 154 sowie in nasaler Richtung angeordnet ist. Als mögliche Erklärung hierfür könnte sich eine Verringerung der Schichtdicke 122 der Aderhaut 124 in den von dem subfovealen Bereich 154 weiter entfernten Bereichen 150 sowie in nasaler Richtung anbieten.

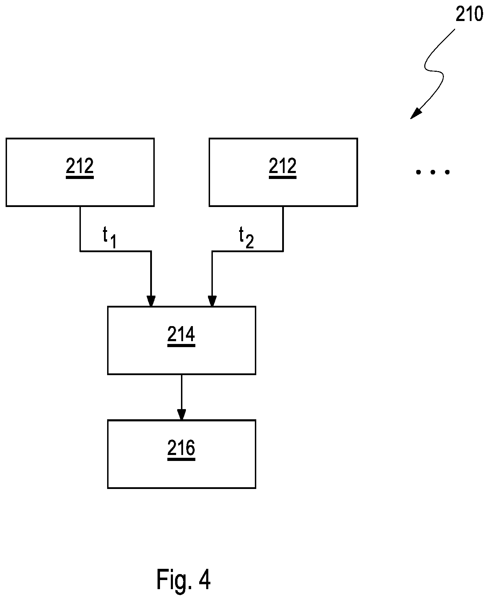

Figur 4 zeigt schematisch ein Ablaufdiagramm eines bevorzugten Ausführungsbeispiels eines erfindungsgemäßen Verfahrens 210 zur Bestimmung der Veränderung des Refraktionsfehlers des Auges 112 des Nutzers 114.

In einem Ermittlungsschritt 212 erfolgt hierzu gemäß Schritt a) das Ermitteln mindestens eines Wertes für die Schichtdicke 122 der Aderhaut 124 des Auges 112 des Nutzers 114 über den mindestens einen Bereich der 150 der Aderhaut 124.

In einem Bestimmungsschritt 214 erfolgt gemäß Schritt b) die Bestimmung des Wertes 216 für die Veränderung des Refraktionsfehlers des Auges 112 des Nutzers 114 ausschließlich aus mindestens zwei Werten für die Schichtdicke 122 der Aderhaut 124, die jeweils zu unterschiedlichen Zeitpunkten t₁, t₂, ... für den mindestens einen Bereich 150 der Aderhaut 124 ermittelt wurden, wobei der mindestens eine Bereich 150 der Aderhaut 124 aus dem nasalen perifovealen Bereich 168 oder dem nasalen parafovealen Bereich 166 der Aderhaut 124 ausgewählt wird.

Damit ermöglicht das vorliegende Verfahren 210 die gewünschte zuverlässige Bestimmung der Veränderung des Refraktionsfehlers des Auges 112 des Nutzers 114 bei einer hohen Wiederholbarkeit der Messergebnisse und erlaubt daher bevorzugt eine zuverlässigere Klassifizierung des Fortschreitens von Fehlsichtigkeit und zuverlässigere Aussagen über den Erfolg einer Intervention zur Verringerung des Fortschreitens der Fehlsichtigkeit.

### Bezugszeichenliste

- 110: Vorrichtung
- 112: Auge
- 114: Nutzer
- 116: Messeinrichtung
- 118: optische Messeinrichtung
- 120: Einrichtung zur optischen Kohärenztomographie
- 122: Schichtdicke
- 124: Aderhaut (Choroidea)
- 126: Lederhaut (Sclera)
- 128: Netzhaut (Retina)
- 130: retinales Pigmentepithel
- 132: Licht
- 134: optische Achse
- 136: reflektiertes Licht
- 138: Gehäuse
- 140: Auswerteinrichtung
- 142: Verbindung
- 144: Prozessor
- 146: Monitor
- 148: Tastatur
- 150: Bereich
- 152: Länge des Augapfels
- 154: subfovealer Bereich
- 156: parafovealer Bereich
- 158: perifovealer Bereich
- 160: Durchmesser
- 162: Innendurchmesser
- 164: Außendurchmesser
- 166: nasaler parafovealer Bereich
- 168: nasaler perifovealer Bereich
- 210: Verfahren zur Bestimmung einer Veränderung eines Refraktionsfehlers eines Auges eines Nutzers
- 212: Ermittlungsschritt
- 214: Bestimmungsschritt
- 216: Wert der Veränderung des Refraktionsfehlers des Auges des Nutzers

## Patentansprüche

1. Verfahren (210) zur Bestimmung einer Veränderung eines Refraktionsfehlers eines Auges (112) eines Nutzers (114), wobei das Auge (112) des Nutzers (114) eine Aderhaut (124) aufweist, wobei das Verfahren (210) die folgenden Schritte umfasst:
a) Ermitteln mindestens eines Wertes für eine Schichtdicke (122) der Aderhaut (124) des Auges (112) des Nutzers (114) über mindestens einen Bereich (150) der Aderhaut (124); und
b) Bestimmen eines Wertes (216) für die Veränderung des Refraktionsfehlers des Auges (112),
wobei das Bestimmen des Wertes (216) für die Veränderung des Refraktionsfehlers des Auges (112) ausschließlich aus mindestens zwei Werten für die Schichtdicke (122) der Aderhaut (124), die jeweils zu unterschiedlichen Zeitpunkten für den mindestens einen Bereich (150) der Aderhaut (124) ermittelt wurden, erfolgt, dadurch gekennzeichntet, dass der mindestens eine Bereich (150) aus einem nasalen perifovealen Bereich (168) oder einem nasalen parafovealen Bereich (166) ausgewählt wird.

2. Verfahren (210) nach dem vorangehenden Anspruch, **dadurch gekennzeichnet, dass** der mindestens eine Bereich (150) aus dem nasalen perifovealen Bereich (168) ausgewählt wird.

3. Verfahren (210) nach einem der vorangehenden Ansprüche, **dadurch gekennzeichnet, dass** in dem mindestens einen Bereich (150) ein Wert für eine durchschnittliche Schichtdicke (122) der Aderhaut (124) ermittelt wird.

4. Verfahren (210) nach einem der vorangehenden Ansprüche, **dadurch gekennzeichnet, dass** aus dem nasalen perifovealen Bereich (168) der Aderhaut ein Teilfeld ausgewählt wird, das einen äußeren Quadranten eines um einen fovealen Bereich (154) zentrierten Ringraums mit einem Innendurchmesser (162) und einem Außendurchmesser (164) umfasst.

5. Verfahren (210) nach einem der vorangehenden Ansprüche, **dadurch gekennzeichnet, dass** das Ermitteln der Werte für die Schichtdicke (122) der Aderhaut (124) mittels Bildverarbeitung einer räumlich aufgelösten Aufnahme des Auges (112) erfolgt.

6. Verfahren (210) nach einem der vorangehenden Ansprüche, **dadurch gekennzeichnet, dass** zu einem Erfassen der Schichtdicke (122) der Aderhaut (124) ein Verfahren, ausgewählt aus einem optischen Verfahren, einem akustischen Verfahren oder einem fotoakustischen Verfahren, eingesetzt wird.

7. Verfahren (210) nach dem vorangehenden Anspruch, **dadurch gekennzeichnet, dass** das optische Verfahren ausgewählt wird aus einem Verfahren zur optischen Kohärenztomographie oder einem adaptiven optischen Verfahren.

8. Verfahren (210) nach dem vorangehenden Anspruch, **dadurch gekennzeichnet, dass** das Verfahren der optischen Kohärenztomographie ausgewählt wird aus einer Fourier-Domain OCT, einer Swept-Source OCT oder einer Time-Domain OCT, oder dass das adaptive optische Verfahren ausgewählt wird aus einem Verfahren zur Bestimmung einer optischen Transmission oder einem Verfahren zur Bestimmung einer optischen Reflektivität.

9. Computerprogramm zur Bestimmung einer Veränderung eines Refraktionsfehlers eines Auges (112) eines Nutzers (114), wobei das Auge (112) des Nutzers (114) eine Aderhaut (124) aufweist, wobei das Computerprogramm dazu eingerichtet ist, die folgenden Verfahrensschritte durchzuführen:
a) Ermitteln mindestens eines Wertes für eine Schichtdicke (122) der Aderhaut (124) des Auges (112) des Nutzers (114) über mindestens einen Bereich (150) der Aderhaut (124); und
b) Bestimmen eines Wertes (216) für die Veränderung des Refraktionsfehlers des Auges (112)
wobei das Bestimmen eines Wertes (216) für die Veränderung des Refraktionsfehlers des Auges (112) ausschließlich aus mindestens zwei Werten für die Schichtdicke (122) der Aderhaut (124), die jeweils zu unterschiedlichen Zeitpunkten für den mindestens einen Bereich (150) der Aderhaut (124) ermittelt wurden, erfolgt, **dadurch gekennzeichnet, dass** der mindestens eine Bereich (150) aus einem nasalen perifovealen Bereich (168) oder einem nasalen parafovealen Bereich (166) ausgewählt wird.

10. Vorrichtung (110) zur Bestimmung einer Veränderung eines Refraktionsfehlers eines Auges (112) eines Nutzers (114), wobei das Auge (112) des Nutzers (114) eine Aderhaut (124) aufweist, wobei die Vorrichtung (110) umfasst:
- eine Messeinrichtung (116) zum Erfassen einer Schichtdicke (122) der Aderhaut (124) des Auges (112) des Nutzers (114); und
- eine Auswerteinrichtung (140) zum Ermitteln von Werten für die Schichtdicke (122) der Aderhaut (124) über mindestens einen Bereich (150) der Aderhaut (124),
wobei die Auswerteinrichtung (140) weiterhin zum Bestimmen eines Wertes (216) für die Veränderung des Refraktionsfehlers des Auges (112) ausschließlich aus mindestens zwei Werten für die Schichtdicke (122) der Aderhaut (124) eingerichtet ist, wobei die Werte für die Schichtdicke (122) der Aderhaut (124) jeweils zu unterschiedlichen Zeitpunkten für den mindestens einen Bereich (150) der Aderhaut (124) ermittelt werden, **dadurch gekennzeichnet, dass** der mindestens eine Bereich (150) aus einem nasalen perifovealen Bereich (168) oder einem nasalen parafovealen Bereich (166) ausgewählt wird.

11. Vorrichtung (110) nach dem vorangehenden Anspruch, **dadurch gekennzeichnet, dass** die Auswerteinrichtung (140) weiterhin dazu eingerichtet ist, über den mindestens einen Bereich (150) einen Wert für eine durchschnittliche Schichtdicke (122) der Aderhaut (140) zu ermitteln.

12. Vorrichtung (110) nach einem der vorangehenden Vorrichtungsansprüche, **dadurch gekennzeichnet, dass** die Auswerteinrichtung (140) weiterhin dazu eingerichtet ist, aus dem nasalen perifovealen Bereich (168) der Aderhaut ein Teilfeld auszuwählen, das einen äußeren Quadranten eines um einen fovealen Bereich (154) zentrierten Ringraums mit einem Innendurchmesser (162) und einem Außendurchmesser (164) umfasst.

13. Vorrichtung (110) nach einem der vorangehenden Vorrichtungsansprüche, **dadurch gekennzeichnet, dass** die Messeinrichtung (116) zum Erfassen einer Schichtdicke der Aderhaut ausgewählt ist aus einer optischen Messeinrichtung (118), einer akustischen Messeinrichtung und einer fotoakustischen Messeinrichtung.

14. Vorrichtung (110) nach dem vorangehenden Anspruch, **dadurch gekennzeichnet, dass** die optische Messeinrichtung (118) ausgewählt ist aus einer Einrichtung (120) zur optischen Kohärenztomographie oder einer Einrichtung zur Durchführung eines adaptiven optischen Verfahrens.

15. Vorrichtung nach dem vorangehenden Anspruch, **dadurch gekennzeichnet, dass** die Einrichtung (120) zur optischen Kohärenztomographie zur Durchführung einer Fourier-Domain OCT, einer Swept-Source OCT oder einer Time-Domain OCT eingerichtet ist, oder dass die Einrichtung zur Durchführung des adaptiven optischen Verfahrens zur Durchführung einer Bestimmung einer optischen Transmission oder einer optischen Reflektivität eingerichtet ist.

## Claims

1. A method (210) for determining a change in a refractive error of an eye (112) of a user (114), the eye (112) of the user (114) having a choroid (124), wherein the method (210) comprises the following steps:
a) ascertaining at least one value for a layer thickness (120) of the choroid (124) of the eye (112) of the user (114) over at least one region (150) of the choroid (124); and
b) determining a value (216) for the change in the refractive error of the eye (112), wherein the value (216) for the change in the refractive error of the eye (112) is determined only from at least two values for the layer thickness (122) of the choroid (124) which were each ascertained at different times for the at least one region (150) of the choroid (124),
**characterized in**
**that** the at least one region (150) is selected from a nasal perifoveal region (168) or a nasal parafoveal region (166).

2. The method (210) as claimed in the preceding claim, **characterized in that** the at least one region (150) is selected from the nasal perifoveal region (168).

3. The method (210) as claimed in any one of the preceding claims, **characterized in that** a value for an average layer thickness (122) of the choroid (124) is ascertained in the at least one region (150).

4. The method (210) as claimed in any one of the preceding claims, **characterized in that** a partial field is selected from the nasal perifoveal region (168) of the choroid, said field comprising an outer quadrant of an annular space with an inner diameter (162) and an outer diameter (164) centered around a foveal region (154).

5. The method (210) as claimed in any one of the preceding claims, **characterized in that** the values for the layer thickness (122) of the choroid (124) are ascertained by means of performing image processing on a spatially resolved recording of the eye (112).

6. The method (210) as claimed in any one of the preceding claims, **characterized in that** a method selected from an optical method, an acoustic method or a photoacoustic method is used to capture the layer thickness (122) of the choroid (124).

7. The method (210) as claimed in the preceding claim, **characterized in that** the optical method is selected from a method for optical coherence tomography or an adaptive optical method.

8. The method (210) as claimed in the preceding claim, **characterized in that** the optical coherence tomography method is selected from a Fourier domain OCT, a swept source OCT or a time domain OCT, or **in that** the adaptive optical method is selected from a method for determining an optical transmission or a method for determining an optical reflectivity.

9. A computer program for determining a change in a refractive error of an eye (112) of a user (114), the eye (112) of the user (114) having a choroid (124), wherein the computer program is configured to carry out the following method steps:
a) ascertaining at least one value for a layer thickness (120) of the choroid (124) of the eye (112) of the user (114) over at least one region (150) of the choroid (124); and
b) determining a value (216) for the change in the refractive error of the eye (112), wherein a value (216) for the change in the refractive error of the eye (112) is determined only from at least two values for the layer thickness (122) of the choroid (124) which were each ascertained at different times for the at least one region (150) of the choroid (124),
**characterized in**
**that** the at least one region (150) is selected from a nasal perifoveal region (168) or a nasal parafoveal region (166).

10. An apparatus (110) for determining a change in a refractive error of an eye (112) of a user (114), the eye (112) of the user (114) having a choroid (124), wherein the apparatus (110) comprises:
- a measuring device (116) for capturing a layer thickness (122) of the choroid (124) of the eye (112) of the user (114); and
- an evaluation device (140) for ascertaining values for the layer thickness (122) of the choroid (124) over at least one region (150) of the choroid (124),
Wherein the evaluation device (140) is furthermore configured to determine a value (216) for the change in the refractive error of the eye (112) only from at least two values for the layer thickness (122) of the choroid (124), wherein the values for the layer thickness (122) of the choroid (124) are each ascertained at different times for the at least one region (150) of the choroid (124),
**characterized in**
**that** the at least one region (150) is selected from a nasal perifoveal region (168) or a nasal parafoveal region (166).

11. The apparatus (110) as claimed in the preceding claim, **characterized in that** the evaluation device (140) is furthermore configured to ascertain a value for an average layer thickness (122) of the choroid (140) over the at least one region (150).

12. The apparatus (110) as claimed in any one of the preceding apparatus claims, **characterized in that** the evaluation device (140) is a furthermore configured to select a partial field from the nasal perifoveal region (168) of the choroid, said field comprising an outer quadrant of an annular space with an inner diameter (162) and an outer diameter (164) centered around a foveal region (154).

13. The apparatus (110) as claimed in any one of the preceding apparatus claims, **characterized in that** the measuring device (116) for capturing a layer thickness of the choroid is selected from an optical measuring device (118), an acoustic measuring device and a photoacoustic measuring device.

14. The apparatus (110) as claimed in the preceding claim, **characterized in that** the optical measuring device (118) is selected from a device (120) for optical coherence tomography or a device for carrying out an adaptive optical method.

15. The apparatus as claimed in the preceding claim, **characterized in that** the device (120) for optical coherence tomography is configured to carry out a Fourier domain OCT, a swept source OCT or a time domain OCT or **in that** the device for carrying out the adaptive optical method is configured to carry out determination of an optical transmission or an optical reflectivity.

## Revendications

1. Procédé (210) pour déterminer une modification d'un champ de réfraction d'un œil (112) d'un utilisateur (114), l'œil (112) de l'utilisateur (114) possédant une choroïde (124), le procédé (210) comprenant les étapes suivantes :
a) identification d'au moins une valeur pour une épaisseur de couche (122) de la choroïde (124) de l'œil (112) de l'utilisateur (114) sur au moins une zone (150) de la choroïde (124) ; et
b) détermination d'une valeur (216) pour la modification du champ de réfraction de l'œil (112),
la détermination de la valeur (216) pour la modification du champ de réfraction de l'œil (112) s'effectuant exclusivement à partir d'au moins deux valeurs de l'épaisseur de couche (122) de la choroïde (124), lesquelles ont été identifiées respectivement à des instants différents pour l'au moins une zone (150) de la choroïde (124),
**caractérisé en ce que**
l'au moins une zone (150) est choisie parmi une zone périfovéale nasale (168) et une zone parafovéale nasale (166) .

2. Procédé (210) selon la revendication précédente, **caractérisé en ce que** l'au moins une zone (150) est choisie parmi une zone périfovéale nasale (168).

3. Procédé (210) selon l'une des revendications précédentes, **caractérisé en ce qu'**une valeur pour une épaisseur de couche (122) moyenne de la choroïde (124) est identifiée dans l'au moins une zone (150).

4. Procédé (210) selon l'une des revendications précédentes, **caractérisé en ce qu'**un champ partiel est sélectionné à partir de la zone périfovéale nasale (168) de la choroïde, lequel comporte un quadrant externe d'un espace annulaire ayant un diamètre intérieur (162) et un diamètre extérieur (164), centré autour d'une zone fovéale (154).

5. Procédé (210) selon l'une des revendications précédentes, **caractérisé en ce que** l'identification des valeurs pour une épaisseur de couche (122) de la choroïde (124) s'effectue au moyen d'un traitement d'image d'un enregistrement avec résolution spatiale de l'œil (112).

6. Procédé (210) selon l'une des revendications précédentes, **caractérisé en ce qu'**en vue d'une acquisition de l'épaisseur de couche (122) de la choroïde (124), un procédé est utilisé, lequel est sélectionné parmi un procédé optique, un procédé acoustique et un procédé photoacoustique.

7. Procédé (210) selon la revendication précédente, **caractérisé en ce que** le procédé optique est sélectionné parmi un procédé de tomographie par cohérence optique et un procédé optique adaptatif.

8. Procédé (210) selon la revendication précédente, **caractérisé en ce que** le procédé de tomographie par cohérence optique est sélectionné parmi une OCT dans le domaine de Fourier, une OCT à source balayée et une OCT dans le domaine temporel, ou **en ce que** le procédé optique adaptatif est sélectionné parmi un procédé de détermination d'une transmission optique et un procédé de détermination d'une réflectivité optique.

9. Programme informatique pour déterminer une modification d'un champ de réfraction d'un œil (112) d'un utilisateur (114), l'œil (112) de l'utilisateur (114) possédant une choroïde (124), le programme informatique étant conçu pour exécuter les étapes de procédé suivantes :
a) identification d'au moins une valeur pour une épaisseur de couche (122) de la choroïde (124) de l'œil (112) de l'utilisateur (114) sur au moins une zone (150) de la choroïde (124) ; et
b) détermination d'une valeur (216) pour la modification du champ de réfraction de l'œil (112),
la détermination d'une valeur (216) pour la modification du champ de réfraction de l'œil (112) s'effectuant exclusivement à partir d'au moins deux valeurs de l'épaisseur de couche (122) de la choroïde (124), lesquelles ont été identifiées respectivement à des instants différents pour l'au moins une zone (150) de la choroïde (124),
**caractérisé en ce que**
l'au moins une zone (150) est choisie parmi une zone périfovéale nasale (168) et une zone parafovéale nasale (166) .

10. Arrangement (110) pour déterminer une modification d'un champ de réfraction d'un œil (112) d'un utilisateur (114), l'œil (112) de l'utilisateur (114) possédant une choroïde (124), l'arrangement (110) comprenant :
- un dispositif de mesure (116) destiné à acquérir une épaisseur de couche (122) de la choroïde (124) de l'œil (112) de l'utilisateur (114) ; et
- un dispositif d'interprétation (140) destiné à déterminer des valeurs pour l'épaisseur de couche (122) de la choroïde (124) sur au moins une zone (150) de la choroïde (124),
le dispositif d'interprétation (140) étant en outre conçu pour déterminer une valeur (216) pour la modification du champ de réfraction de l'œil (112) exclusivement à partir d'au moins deux valeurs de l'épaisseur de couche (122) de la choroïde (124), les valeurs de l'épaisseur de couche (122) de la choroïde (124) ayant été identifiées respectivement à des instants différents pour l'au moins une zone (150) de la choroïde (124),
**caractérisé en ce que**
l'au moins une zone (150) est choisie parmi une zone périfovéale nasale (168) et une zone parafovéale nasale (166) .

11. Arrangement (110) selon la revendication précédente, **caractérisé en ce que** le dispositif d'interprétation (140) est en outre conçu pour identifier une valeur pour une épaisseur de couche (122) moyenne de la choroïde (140) dans l'au moins une zone (150).

12. Arrangement (110) selon l'une des revendications précédentes concernant l'arrangement (110), **caractérisé en ce que** le dispositif d'interprétation (140) est en outre conçu pour sélectionner un champ partiel à partir de la zone périfovéale nasale (168) de la choroïde, lequel comporte un quadrant externe d'un espace annulaire ayant un diamètre intérieur (162) et un diamètre extérieur (164), centré autour d'une zone fovéale (154).

13. Arrangement (110) selon l'une des revendications précédentes concernant l'arrangement (110), **caractérisé en ce que** le dispositif de mesure (116), en vue d'une acquisition d'une épaisseur de couche de la choroïde, est sélectionné parmi un dispositif de mesure optique (118), un dispositif de mesure acoustique et un dispositif de mesure photoacoustique.

14. Arrangement (110) selon la revendication précédente, **caractérisé en ce que** le dispositif de mesure optique (118) est sélectionné parmi un dispositif (120) de tomographie par cohérence optique et un dispositif destiné à mettre en œuvre un procédé optique adaptatif.

15. Arrangement selon la revendication précédente, **caractérisé en ce que** le dispositif (120) de tomographie par cohérence optique est conçu pour mettre en œuvre une OCT dans le domaine de Fourier, une OCT à source balayée ou une OCT dans le domaine temporel, ou **en ce que** le dispositif destiné à mettre en œuvre le procédé optique adaptatif est conçu pour effectuer une détermination d'une transmission optique ou d'une réflectivité optique.
